(19) Europäisches Patentamt
European Patent Office
Office européen des brevets

(11) Numéro de publication: 0 070 218
B1

(12) # FASCICULE DE BREVET EUROPEEN

(45) Date de publication du fascicule du brevet: **19.06.85**

(51) Int. Cl.[4]: **C 07 C 25/18**, C 08 K 5/03 // C07C17/12

(21) Numéro de dépôt: **82401199.3**

(22) Date de dépôt: **29.06.82**

(54) **Composés aromatiques halogénés pour ignifugation.**

(30) Priorité: **10.07.81 FR 8113600**

(43) Date de publication de la demande: **19.01.83 Bulletin 83/3**

(45) Mention de la délivrance du brevet: **19.06.85 Bulletin 85/25**

(84) Etats contractants désignés: **AT BE CH DE FR GB IT LI NL SE**

(56) Documents cités:
**DE - A - 2 758 781**
**FR - A - 2 207 105**

(73) Titulaire: **ATOCHEM, 12/16, allée des Vosges, F-92400 Courbevoie (FR)**

(72) Inventeur: **Bernard, Michel Paul Charles, 90, rue Marius Aufan, F-92300 Levallois (FR)**
Inventeur: **Mathais, Henri Hameau de Saint-Didier, Villa No 2 Chemin de l'Indiennerie, F-69370 Saint-Didier au Mont d'Or (FR)**
Inventeur: **Gagnieur, André Raymond, 13, rue Cornillon Fontanil, F-38120 Saint-Egreve (FR)**

(74) Mandataire: **Rochet, Michel et al, ATOCHEM Département Propriété Industrielle Cédex 22, F-92091 Paris la Défense (FR)**

**EP 0 070 218 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux composés aromatiques halogénés présentant des qualités d'ignifugation pour les matières synthétiques.

De nombreux produits, notamment halogénés, ont été proposés comme ignifugeants des matières synthétiques. Cepentant, ces produits exercent généralement des effets secondaires nuisibles en altérant les propriétés caractéristiques des matériaux auxquels ils sont incorporés, en particulier en les rendant plus fragiles.

Le brevet US n° 3 403 036 décrit des compositions de polymères ignifugés ayant des propriétés mécaniques convenables, qui contiennent comme agents d'amélioration de la tenue au feu des produits de di-addition d'hexahalogénocyclopentadiène sur un composé cyclique insaturé. Mais en téalité les seuls produits décrits et exemplifiés sont des dérivés chlorés qui sont connus pour être moins efficaces que les produits bromés en tant qu'ignifugeants.

Le brevet français FR 2 301 556 décrit des compositions de matières plastiques dans lesquelles l'efficacité des dérivés chlorés précédents est renforcée par l'addition d'un aromatique bromé connu comme ignifugeant. Cette association n'est qu'un compromis entre l'amélioration de la réaction au feu et la conservation de propriétés mécaniques et physiques acceptables.

Le DE-A 2 758 781 décrit des dérivés polybenzéniques halogénés, dans lesquels les noyaux benzéniques sont liés les uns aux autres par un radical = CHCl, = CHBr ou = CHOH. Ces produits, du fait de la présence sur la chaîne aliphatique d'halogène ou de function hydroxyle, peuvent présenter une certaine instabilité entrainant une coloration prononcée des résines plastiques, notamment des copolymères acrylonitrile-butadiène-styrène.

Quant aux brevets US 3 862 et 4 016 137 ils décrivent des compositions de matières synthétiques contenant des composés de bisphénoxy bromés, dont l'inconvénient pour cet usage est d'avoir une faible teneur en brome, ce qui nécessite des taux d'ajoute importants si l'on veut obtenir une amélioration valable du comportement au feu des compositions obtenues.

Un grand nombre d'ignifugeants aromatiques bromés sont également connus, mais qui ont l'inconvénient de donner lieu dans certaines matières plastiques à des phénomènes de migration en surface au cours du temps. Cette efflorescence constitue un problème sérieux puisqu'elle détériore l'aspect de l'objet fini.

La demanderesse a mis au point de nouveaux dérivés aromatiques halogénés qui ne présentent pas ces inconvénients. Ils ont la formule générale suivante:

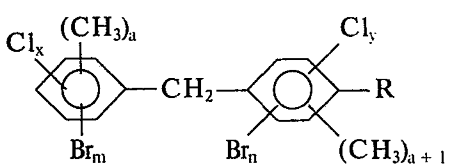

dans laquelle R est un atome d'hydrogène ou m groupement bromobenzyle, éventuellement chloré et/ou méthylé

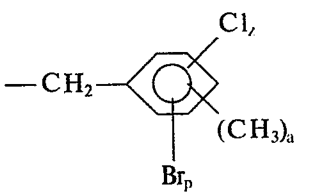

x, y et z pouvant avoir la valeur de 0 à 2, a pouvant être égal à 0 ou 1, la somme (m + n + 2 a) étant comprise entre 3 et 9 et la somme (m + n + p + 3 a) étant comprise entre 4 et 13, la teneur globale en halogènes étant supérieure à 65% en poids.

Lorsque R représente un atome d'hydrogène, on a des benzyltoluènes polybromés et éventuellement substitués par du chlore et/ou un ou plusieurs groupes méthyle. Lorsque R représente un groupement benzyle, substitué ou non, on a des bis-(benzyle) toluènes polybromés et éventuellement substitués par du chlore et/ou un ou plusieurs groupes méthyle.

Ces produits sont obtenus par une bromation plus ou moins poussée des benzyltoluènes, substitués ou non, selon les valeurs de x, y, et a de la formule suivante:

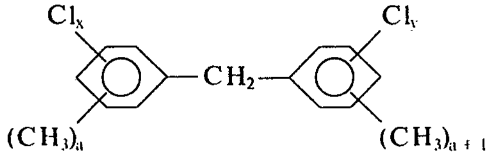

et des bis-(benzyle) toluènes, substitués ou non selon les valeurs de x, y, et a de la formule suivante:

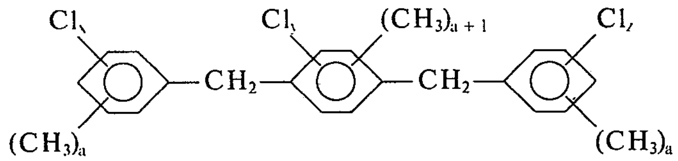

On peut obtenir également un mélange de composés bromés par une bromation d'un mélange des benzyltoluènes et des bis-(benzyle) toluènes précédents.

Les composés aromatiques halogénés de l'invention sont des agents d'amélioration du comportement au feu de nombreuses matières de synthèse naturellement inflammables. Parmi celles-ci, on peut citer les homopolymères et copolymères de chlorure de vinyle, d'éthylène, de propylène, de styrène, d'acrylonitrile, de butadiène — c'est-à-dire les PVC, rigides et plastifiés, les polyoléfines, polystyrènes, résines ABS, les thermoplastiques polycondensés comme les polyesters saturés et les polyamides, les thermodurcissables comme les polyesters insaturés, les polyépoxydes, les polyuréthannes, les polyacrylates, et les caoutchoucs et élastomères.

Les composés de l'invention présentent une grande efficacité pour l'amélioration du comportement au feu des compositions dans lesquelles ils sont incorporés. Ceci est dû à leur teneur élevée en halogènes, supérieure à 65% en poids, et le cas échéant à l'association chlore/brome. De plus, ils sont faciles à incorporer dans les matières synthétiques sans dégradation ou décomposition au cours des opérations de mélangeage ou de mise en oeuvre, et n'ont pas tendance à migrer en surface.

Les matières synthétiques traitées par ces additifs ont une réaction au feu nettement améliorée et gardent des propriétés physiques et mécaniques satisfaisantes, notamment la résistance au choc.

Les composés de l'invention sont introduits dans les compositions de matières synthétiques à raison de 2 à 40% en poids et de préférence de 4 à 25% en poids de la composition finale obtenue.

Ils peuvent être utilisés en association avec des produits de renforcement de l'effet ignifugeant comme des dérivés de l'antimoine, de l'arsenic, du bismuth, de l'étain et du zinc, notamment le trioxyde d'antimoine, ces dérivés étant introduits à raison de 1 à 25% en poids de la composition finale obtenue.

L'utilisation des produits de l'invention n'exclut pas l'introduction éventuelle des adjuvants habituels comme les promoteurs d'adhérence, les anti-oxydants, les agents antistatiques, les lubrifiants, les pigments, les stabilisants thermiques, les charges, etc.

Leur incorporation dans les matières synthétiques a lieu selon les techniques habituelles, par exemple celles décrites dans le brevet français n° 2 459 264.

Les exemples suivants illustrent l'invention sans toutefois la limiter.

## Exemple 1

### Synthèse de chlorobenzylchlorotoluène polybromé (CBCTB)

$$C_{12}H_{[7-(m+n)]}Br_{(m+n)}Cl_2C_2H_5$$

Dans un réacteur en verre d'une capacité de 1 litre équipé d'un agitateur, d'une prise de température, d'une ampoule de coulage, d'une colonne de distillation surmontée d'un réfrigérant à reflux et d'un dispositif permettant de recuillir l'acide bromhydrique dégagé, on charge 1120 g de brome (7 moles) et 5 g de chlorure ferrique comme catalyseur.

En deux heures et demie, on introduit progressivement sous agitation 125,5 g (0,5 mole) de chlorobenzyl-chlorotoluène (CBCT) de formule:

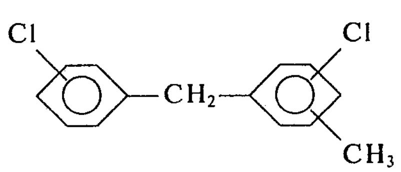

préalablement obtenu suivant la méthode décrite dans le brevet français n° 2 432 199, en maintenant la température du milieu à 55—60°C de manière à assurer un léger reflux de brome.

Le mélange réactionnel est maintenu sous agitation et à reflux pendant cinq heures jusqu'à récupération d'une quantité d'environ 265 g d'acide bromhydrique HBr. La quantité de brome entraînée par cet HBr est d'environ 70 g.

A ce stade la suspension dans le brome liquide de CBCTB est refroidie à 40°C et additionnée de 3 g d'eau dans le but de détruire le catalyseur. On ajoute 600 g de dibromoéthane et porte le mélange à

ébullition ce qui permet de séparer par distillation 440 g de brome jusqu'à une température de 120° C en pied.

Le brome restant dans le réacteur est transformé en dibromoéthane par injection dans le milieu à 20—30°C d'éthylène pendant environ 1 h 30, à un débit de 20 l/h.

La suspension de monochlorobenzylmonochlorotoluène bromé dans le dibromoéthane est lavée par une solution d'acide chlorhydrique à 5% une première fois à l'ébullition et une deuxième fois à température ambiante puis par l'eau à plusieurs reprises à température ambiante ou éventuellement vers 50° C pour faciliter la décantation.

Le monochlorobenzylmonochlorotoluène bromé est séparé par filtration sur un verre fritté puis lavé à l'eau sur filtre et essoré.

Par séchage à 80° C sous pression réduite on obtient 330 g de monochlorobenzylmonochlorotoluène bromé de point de fusion 220—240°C. La teneur en brome déterminée par analyse est de 68,1%, ce qui correspond à un nombre moyen (m + n) d'atomes de brome substituant de 6,5 par molécule.

## Exemple 2

On procède dans les mêmes conditions que dans l'exemple 1 en introduisant 125,5 g (0,5 mole) de monochlorobenzylmonochlorotoluène en 5 heures dans le brome à reflux contenant du chlorure ferrique. On maintient ensuite le mélange réactionnel sous agitation et à reflux pendant 2 heures jusqu'à récupération d'une quantité d'environ 250 g d'acide bromhydrique HBr.

La suite de l'opération est conduite comme dans l'exemple 1 et on recueille finalement 320 g de monochlorobenzylmonochlorotoluène bromé de point de fusion 206—230°C. La teneur en brome déterminée par analyse est de 65,4%, ce qui correspond à un nombre moyen m + n d'atomes de brome substituant de 5,8 par molécule.

## Exemple 3

Synthèse de bis(monochlorobenzyle) monochlorotoluène polybromé (BCBCTB)

$$C_{18}H_{[10-(m+n+p)]}Br_{(m+n+p)}Cl_3C_3H_7$$

On procède de la même manière que dans l'exemple 1 en chargeant dans le réacteur 1400 g (8,75 moles) de brome et 5 g de chlorure d'aluminium comme catalyseur.

En deux heures, on introduit progressivement 187,5 g (0,5 mole) de bis(monochlorobenzyle) monochlorotoluène de formule générale:

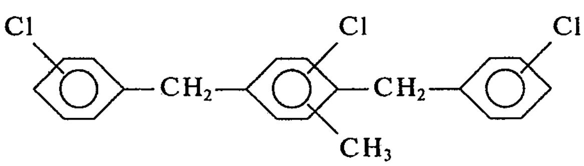

Ce composé est un sous-produit isolé de la préparation du monochlorobenzylmonochlorotoluène, comme indiqué dans le brevet français n° 2 432 199.

Le mélange réactionnel est maintenu sous agitation à reflux pendant 6 heures jusqu'à récupération d'une quantité d'environ 325 g d'acide bromhydrique HBr. La quantité de brome entrainée par cet HBr est d'environ 85 g.

Après refroidissement de la suspension dans le brome liquide et addition d'eau, on ajoute 900 g de dibromoéthane.

La quantité de brome récupérée par distillation est de 550 g.

La suite de l'opération est conduite comme dans l'exemple 1 et on recueille finalement 375 g de bis-(monochlorobenzyle) monochlorotoluène bromé de point de fusion 200—280°C.

La teneur en brome déterminée par analyse est de 63,5%, ce qui correspond à un nombre moyen m + n + p d'atomes de brome substituant de 8 par molécule.

## Exemple 4

Synthèse de benzyltoluène polybromé

$$C_{12}H_{[9-(m+n)]}Br_{(m+n)}C_2H_5$$

On procède selon l'exemple 1 en chargeant dans le réacteur de 1 litre 1120 g de brome (7 moles) et 1,25 g de chlorure ferrique.

Le benzyltoluène de formule

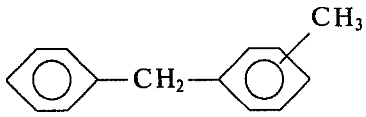

est introduit progressivement en 1 h 45 en maintenant la température à 45°C.

On maintient sous agitation environ 0 h 15 pour obtenir 290 g d'acide bromhydrique entraînant environ 60 g de brome.

La suite de l'opération est conduite selon l'exemple 1 en ajoutant 1 g d'eau et 660 g de dibromoéthane. On distille 410 g de brome jusqu'à 120°C en pied. On injecte en 1 h 15 de l'éthylène à un débit de 40 l/h.

Après filtration et lavage, on obtient 230 g de benzyltoluène bromé de point de fusion 190—200°C et de teneur en brome analysée de 77 % correspondant à m + n = 7,3 atomes de brome par molécule.

### Exemple 5

Synthèse de dichlorobenzyldichlorotoluène polybromé

$$C_{12}H_{[5-(m+n)]}Cl_4Br_{(m+n)}C_2H_5$$

On procède selon l'exemple 1 en chargeant dans le réacteur 960 g de brome (6 moles), 5 g de chlorure ferrique, puis 160 g (0,5 mole) de dichlorobenzyldichlorotoluène de formule

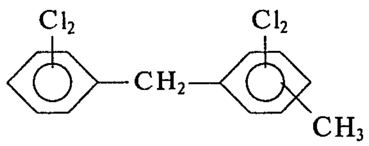

en 2 h 45 minutes, tout en maintenant la température à 55—60°C. On maintient à cette température et sous agitation pendant environ 4 heures pour dégager 195 g d'acide bromhydrique contenant environ 50 g de brome. On ajoute 3 g d'eau et 660 g de dibromoéthane. On distille 450 g de brome jusqu'à 110°C en pied. On injecte de l'éthylène à 20 l/h pendant environ 1 h 30.

Après filtration et lavage, on recueille 290 g de dichlorobenzyldichlorotoluène bromé de point de fusion 260—275°C. La teneur en brome déterminée par analyse est de 53,2 %, ce qui correspond à m + n = 4,5 atomes de brome par molécule.

### Exemple 6

Synthèse de xylylxylène polybromé

$$C_{12}H_{[7,13-(m+n)]}Br_{(m+n)}C_4H_{10,87}$$

On procède selon l'exemple 1 en chargeant dans un réacteur de 2 litres 2240 g (14 moles) de brome et 2,5 g de chlorure ferrique comme catalyseur.

En 2 h 20 minutes, on introduit régulièrement, en maintenant la température à 30°C, 210 g (1 mole) de xylylxylène industriel de composition:

87 % 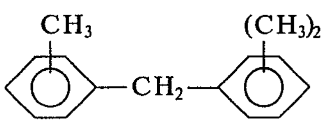

13 % 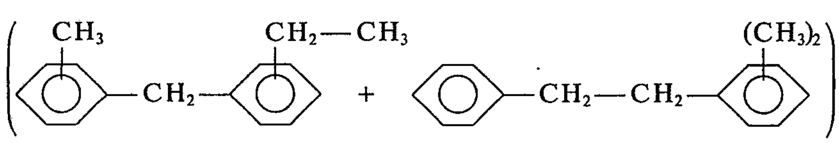

On refroidit ensuite à 20°C en 0 h 15 en dégazant à l'azote; la quantité d'acide bromhydrique absorbée est de 515 g contenant environ 85 g de brome.

On poursuit l'opération selon l'exemple 1 en ajoutant 1,5 g d'eau et 850 g de dibromoéthane. On distille 640 g de brome jusqu'à 100°C en pied, avec simultanément dégagement d'environ 40 g de HBr. On injecte de l'éthylène à 60 l/h en 2 heures.

On recueille 640 g de xylylxylène bromé de point de fusion 140–200°C et de teneur en brome total 72,5%, ce qui correspond pour m + n + 2a à environ 8,7.

Exemple 7

Cet exemple montre les possibilités d'application des produits de l'invention.

Une résine d'acrylonitrile-butadiène-styrène (ABS) de marque UGIKRAL SF de PCUK – Produits Chimiques Ugine Kuhlmann est utilisée pour préparer des éprouvettes contenant les différentes compostions décrites dans le tableau 1.

Ces compositions sont préparées par le mélange des constituants à 160°C sur un mélangeur à cylindres pendant environ 10 minutes. La matière obtenue en fin d'opération se présente sous la forme d'une feuille de faible épaisseur qui est ensuite transformée en granulés, ceux-ci étant alors transformés en barreaux de 127 × 127 × 3,2 mm au moyen d'une presse à injecter, à la température de 230°C.

On incorpore dans ces compositions du monochlorobenzylmonochlorotoluène bromé préparé dans les conditions de l'exemple 1, du bis-(monochlorobenzyle) monochlorotoluène bromé préparé dans les conditions de l'exemple 3 ou du benzyltoluène bromé préparé dans les conditions de l'exemple 4.

Les mélanges contiennent également du trioxyde d'antimoine $Sb_2O_3$ (qualité pour ignifugation).

Deux éprouvettes sont également préparées à titre comparatif en remplaçant les dérivés de l'invention soit par du décabromodiphényle éther soit par du bis (tribromophénoxy)-1,2 éthane.

Sur ces éprouvettes on a déterminé l'indice d'oxygène selon la norme française T 51-071. Plus cet indice est élevé, meilleure est la réaction au feu de la compositions étudiée.

La réaction au feu a été également évaluée selon la méthode des Underwriters' Laboratories UL 94 décrite dans »Standard for tests for Flammability of Plastics for parts in devices and appliances materials«, seconde édition, mai 1975. Le résultat de l'essai vertical UL 94 est évalué par un classement des réactions au feu qui du moins bon au meilleur va de »non classé« à V 2, V 1 et V0.

Le tableau 1 présente les résultats des essais de réaction au feu et de résistance au choc mesurés pour les différentes compositions.

Tableau 1

| Compositions (parties en poids) | I | II | III | IV | V | VI |
|---|---|---|---|---|---|---|
| Polymère ABS, UGIKRAL SF | 100 | 100 | 100 | 100 | 100 | 100 |
| Monochlorobenzylmonochlorotoluène bromé | – | 18 | – | – | – | – |
| Bis-(monochlorobenzyle) monochlorotoluène bromé | – | – | 18 | – | – | – |
| Benzyltoluène bromé | – | – | – | 18 | – | – |
| Décabromodiphényle éther | – | – | – | – | 18 | – |
| Bis-(tribromophenoxy)-1,2 éthane | – | – | – | – | – | 18 |
| $Sb_2O_3$ | – | 6 | 6 | 6 | 6 | 6 |
| Indice d'oxygène selon Norme AFNOR NFT 51-071 (%) | 18 | 25,3 | 26,3 | 26,7 | 25,0 | 23,5 |
| Classement selon UL 94 (épaisseur des éprouvettes: 3,2 mm) | Non classé | V0 | V0 | V0 | V0 | Non classé |
| Résistance au choc IZOD entaillé à 23°C selon ISO R 180 (J/m d'entaille) | 300 | 150 | 100 | 155 | 55 | 150 |

Des éprouvettes des compositions II, III, IV et VI du tableau 1 sont placées en étuve à 120°C pendant 24 heures. Dans ces conditions, l'éprouvette VI contenant le bis-(tribromophénoxy)-1,2 éthane montre l'apparition d'une efflorescence en surface.

Ce phénomène n'apparaît pas pour les éprouvettes II, III et IV.

Exemple 8

On prépare dans des conditions analogues à celle de l'exemple 7 des compositions d'ABS contenant différents taux de monochlorobenzylchlorotoluène bromé préparé dans les conditions de l'exemple 1, ou de benzyltoluène bromé préparé dans les conditions de l'exemple 4, en mélange avec différentes teneurs en $Sb_2O_3$.

A titre comparatif, une composition est préparée en remplaçant les produits de l'invention par de l'octabromodiphényle éther.

Le tableau 2 présente les résultats des essais de réaction au feu et de résistance au choc mesurés pour ces compositions.

Tableau 2

| Compositions (parties en poids) | VII | VIII | IX | X | XI | XII |
|---|---|---|---|---|---|---|
| Polymère ABS, UGIKRAL SF | 100 | 100 | 100 | 100 | 100 | 100 |
| Monochlorobenzylmonochloro-toluène bromé | 15 | 17 | 20 | 30 | – | – |
| Benzyltoluène bromé | – | – | – | – | 15 | – |
| Octabromodiphényle éther | – | – | – | – | – | 17 |
| $Sb_2O_3$ | 5 | 7 | 10 | 10 | 5 | 7 |
| Indice d'oxygène selon NFT 51-071 (%) | 23,6 | 25,3 | 27,5 | 30,7 | 24,8 | 25,0 |
| Classement selon UL 94 (épaisseur éprouvettes: 3,2 mm) | V2 | V0 | V0 | V0 | V2 | V0 |
| Résistance au choc IZOD entaillé à 23°C (J/m d'entaille) | 160 | 145 | 120 | 98 | 171 | 110 |

Exemple 9

Une résine de polystyrène »choc« obtenue par mélange en proportions 50 : 50 de perles de polystyrène de qualité »cristal« et de qualité »hyperchoc« et contenant globalement 5 % de polybutadiène est utilisée comme base à différentes compositions de thermoplastiques.

Les conditions de transformation et de préparation des éprouvettes sont semblables à celles décrites dans l'exemple 7.

La préparation sur mélangeur à cylindres s'effectue à 150°C et l'injection à 190°C.

Ces compositions contiennent différents taux de monochlorobenzylmonochlorotoluène bromé préparé dans les conditions de l'exemple 1 ou de benzyltoluène bromé préparé dans les conditions de l'exemple 4, en mélange avec différentes teneurs en $Sb_2O_3$.

Deux compositions sont préparées à titre comparatif en remplaçant le produit de l'invention par du décabromodiphényle éther. Les résultats des essais de réaction au feu et de résistance au choc mesurés pour ces compositions sont présentés dans le tableau 3.

Tableau 3

| Compositions (parties en poids) | XIII | XIV | XV | XVI | XVII | XVIII | XIX | XX | XXI |
|---|---|---|---|---|---|---|---|---|---|
| Polystyrènechoc*) | 100 | 100 | 100 | 100 | 100 | 100 | 100 | 100 | 100 |
| Monochlorobenzylmonochlorotoluène bromé | – | 9 | 12 | 15 | 20 | 30 | – | – | – |
| Benzyltoluène bromé | – | – | – | – | – | – | 15 | – | – |
| Decabromodiphényle éther | – | – | – | – | – | – | – | 15 | 20 |
| $Sb_2O_3$*) | – | 3 | 6 | 5 | 10 | 10 | 5 | 5 | 10 |
| Indice d'oxygène selon NFT 51-071 (%) | 17,5 | 21,0 | 22,5 | 26,0 | 28,0 | 32,0 | 25,0 | 24,7 | 27,5 |
| Classement selon UL 94 (épaisseur éprouvettes: 3,2 mm) | Non Classé | V2 | V2 | V0 | V0 | V0 | V0 | V0 | V0 |
| Résistance au choc IZOD entaillé à 23°C (J/m d'entaille) | 110 | 80 | 75 | 70 | 56 | 37 | 85 | 54 | 50 |

*) mélange 50 : 50: – polystyrène »cristal«,
– polystyrène »hyperchoc« à 10% de polybutadiène.

Exemple 10

Le monochlorobenzylmonochlorotoluène bromé de l'exemple 1, le benzyltoluène bromé de l'exemple 4, le dichlorobenzyldichlorotoluène bromé de l'exemple 5 et le xylylxylène bromé de l'exemple 6 sont introduits à différents taux dans une résine de polyéthylène, type haute densité (melt index à 230°C sous 5 kg: 18) et dans une résine de polypropylène (melt index à 230°C sous 5 kg: 6).

Les conditions de transformation et de préparation sont semblables à celles décrites dans l'exemple 7. Pour le polyéthylène Hd l'incorporation sur mélangeur à cylindres s'effectue à 175°C et l'injection à 170°C. Pour le polypropylène les températures correspondantes sont respectivement de 160 et 180°C. Les compositions contiennent également du trioxyde d'antimoine à différentes teneurs. Les résultats des essais de réaction au feu et de résistance au choc mesurés pour ces compositions sont présentés dans le tableau 4.

Tableau 4

| Compositions (parties en poids) | XXII | XXIII | XXIV | XXV | XXVI | XXVII | XXVIII | XXIX | XXX | XXXI |
|---|---|---|---|---|---|---|---|---|---|---|
| Polyéthylène Hd | 100 | 100 | 100 | 100 | 100 | 100 | 100 | – | – | – |
| Polypropylène | – | – | – | – | – | – | – | 100 | 100 | 100 |
| Monochlorobenzylmonochlorotoluène bromé | – | 20 | 30 | 45 | – | – | – | – | 45 | – |
| Benzyltoluène bromé | – | – | – | – | 20 | – | – | – | – | – |
| Dichlorobenzyldichlorotoluène bromé | – | – | – | – | – | 20 | – | – | – | 51 |
| (Méthylbenzyle)xylène bromé | – | – | – | – | – | – | 20 | – | – | – |
| $Sb_2O_3$ | – | 7 | 10 | 15 | 7 | 7 | 7 | – | 15 | 17 |
| Indice d'oxygène selon NFT 51-071 (%) | 17,0 | 24,0 | 25,0 | 25,3 | 24,7 | 24,5 | 24,8 | 18,0 | 25,0 | 25,5 |
| Classement selon UL 94 (épaisseur éprouvettes: 3,2 mm) | Non Classé | V 2 | V 2 | V 0 | V 2 | V 2 | V 2 | Non Classé | V 2 | V 2 |
| Résistance au choc IZOD entaillé à 23°C (J/m d'entaille) | 55 | 33 | 32 | 26 | 24 | 22 | 24 | 45 | 23 | 23 |

Exemple 11

Le monochlorobenzylmonochlorotoluène bromé de l'exemple 1 et le benzyltoluène bromé de l'exemple 4 sont introduits à différents taux dans une résine de polychlorure de vinyle EKAVYL SK 55 de PCUK — Produits Chimiques Ugine Kuhlmann.

Les conditions de préparation des compositions sont analogues à celles décrites dans l'exemple 7 (incorporation sur mélangeur à cylindres à 165°C. Injection à 170°C).

Les compositions contiennent également du trioxyde d'antimoine à différentes teneurs.

Une composition est préparée, à titre comparatif, en remplaçant les produits de l'invention par du décabromodiphényle éther.

Les résultats des essais de réaction au feu et de résistance au choc mesurés pour ces compositions sont présentés dans le tableau 5.

Tableau 5

| Compositions (parties en poids) | XXXII | XXXIII | XXXIV | XXXV | XXXVI |
|---|---|---|---|---|---|
| Polychlorure de vinyle | 100 | 100 | 100 | 100 | 100 |
| Lubrifiants et stabilisants | 5 | 5 | 5 | 5 | 5 |
| Monochlorobenzylmonochloro-toluène bromé | — | 6 | 10 | — | — |
| Benzyltoluène bromé | — | — | — | 6 | — |
| Decabromodiphényle éther | — | — | — | — | 6 |
| $Sb_2O_3$ | — | 3 | 5 | 3 | 3 |
| Indice d'oxygène selon NFT 51-071 (%) | 40,0 | 47,0 | 48,0 | 49,0 | 47,0 |
| Classement selon UL 94 (épaisseur des éprouvettes: 3,2 mm) | VO | VO | VO | VO | VO |
| Résistance au choc IZOD entaillé à 23°C (J/m d'entaille) | 51 | 44 | 34 | 44 | 44 |

**Revendications**

1. Produits halogénés ayant la formule générale suivante:

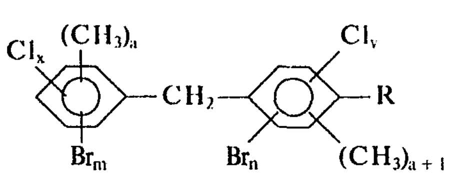

dans laquelle R est un atome d'hydrogène ou un groupement bromobenzyle éventuellement substitué par du chlore et/ou un groupement méthyle

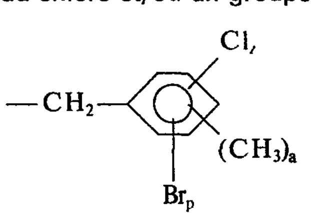

x, y et z pouvant avoir la valeur de 0 à 2, a pouvant être égal à 0 ou 1, la somme (m + n + 2 a) étant comprise entre 3 et 9, la somme (m + n + p + 3 a) étant comprise entre 4 et 13 et la teneur en halogènes étant supérieure à 65% en poids de la molécule.

2. Produits bromés selon la revendication 1 dans lesquels R représente un atome d'hydrogène et x, y et a sont nuls.

3. Produits bromés selon la revendication 1 dans lesquels R représente un atome d'hydrogène, x et y sont égaux à 1 et a est nul.

4. Produits bromés selon la revendication 1 dans lesquels R représente un atome d'hydrogène, x et y sont égaux à 2 et a est nul.

5. Produits bromés selon la revendication 1 dans lesquels R représente un atome d'hydrogène, x et y sont nuls et a est égal à 1.

6. Produits bromés selon la revendication 1 dans lesquels R représente un groupement bromobenzyle et x, y et a sont nuls.

7. Produits bromés selon la revendication 1 dans lesquels R représente un groupement monochlorobromobenzyle, x et y sont égaux à 1 et a est nul.

8. Application des produits de la revendication 1 à l'ignifugation des matières synthétiques.

9. Application selon la revendication 8 dans laquelle les produits de la revendication 1 sont introduits dans les matières synthétiques à ignifuger, à raison de 2 à 40% en poids de la compositon finale obtenue.

10. Application selon la revendication 9 contenant du trioxyde d'antimoine à teneur de 1 à 25% en poids de la composition finale obtenue.

11. Compositions de matières synthétiques ignifugées selon l'une des revendications 8 à 10.

**Patentansprüche**

1. Halogenhaltige Produkte der allgemeinen Formel

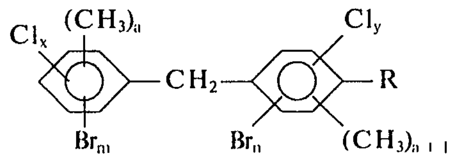

in der R ein Wasserstoffatom oder eine Brombenzylgruppe, die gegebenenfalls durch Chlor und/oder eine Methylgruppe substituiert ist, bedeutet

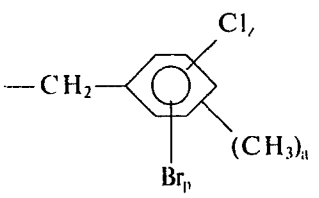

wobei x, y und z Werte von 0 bis 2 haben können, a 0 oder 1 sein kann, die Summe (m + n + 2 a) Werte von 3 bis 9, die Summe (m + n + p + 3 a) Werte von 4 bis 13 annehmen kann, und wobei der Halogengehalt über 65% des Molekulargewichts beträgt.

2. Bromhaltige Produkte nach Anspruch 1, in denen R ein Wasserstoffatom ist und x, y und a 0 sind.

3. Bromhaltige Produkte nach Anspruch 1, in denen R ein Wasserstoffatom ist, x und y = 1 sind und a = 0 ist.

4. Bromhaltige Produkte nach Anspruch 1, in denen R ein Wasserstoffatom ist, x und y = 2 sind und a = 0 ist.

5. Bromhaltige Produkte nach Anspruch 1, in denen R ein Wasserstoffatom ist, x und y = 0 sind und a = 1 ist.

6. Bromhaltige Produkte nach Anspruch 1, in denen R eine Brombenzylgruppe ist und x, y und a = 0 sind.

7. Bromhaltige Produkte nach Anspruch 2, in denen R eine Monochlorbrombenzylgruppe ist, x und y = 1 sind und a = 0 ist.

8. Anwendung der Produkte nach Anspruch 1 zum Flammfestmachen von Kunststoffen.

9. Anwendung nach Anspruch 8, in der Produkte nach Anspruch 1 in Mengen von 2 bis 40 Gew.% der erhaltenen Endzusammensetzung zum Flammfestmachen in Kunststoffe eingebracht werden.

10. Anwendung nach Anspruch 9, wobei die erhaltene Endzusammensetzung Antimontrioxid in einer Menge zwischen 1 und 25 Gew.% enthält.

11. Zusammensetzungen von flammfest gemachten Kunststoffen nach einem der Ansprüche 8 bis 10.

## Claims

1. Halogenated products having the following general formula:

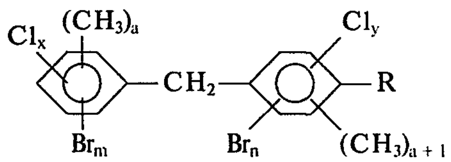

in which R is a hydrogen atom or a bromobenzyl group optionally substituted by chlorine and/or a methyl group

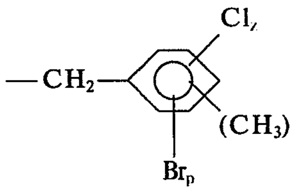

x, y and z being capable of having a value from 0 to 2, a being capable of being equal to 0 or 1, the sum (m + n + 2 a) being between 3 and 9, the sum (m + n + p + 3 a) being between 4 and 13 and the content of halogens being greater than 65 % by weight of the molecule.

2. Brominated products according to Claim 1, in which R denotes a hydrogen atom and x, y and a are zero.

3. Brominated products according to Claim 1, in which R denotes a hydrogen atom, x and y are equal to 1 and a is zero.

4. Brominated products according to Claim 1, in which R denotes a hydrogen atom, x and y are equal to 2 and a is zero.

5. Brominated products according to Claim 1, in which R denotes a hydrogen atom, x and y are zero and a is equal to 1.

6. Brominated products according to Claim 1, in which R denotes a bromobenzyl group and x, y and a are zero.

7. Brominated products according to Claim 1, in which R denotes a monochlorobromobenzyl group, x and y are equal to 1 and a is zero.

8. Application of the products of Claim 1 to flameproofing of synthetic substances.

9. Application according to Claim 8, in which the products of Claim 1 are introduced into the synthetic substances to be flameproofed, in a proportion of 2 to 40 % by weight of the final composition obtained.

10. Application according to Claim 9, containing antimony trioxide at a concentration of 1 to 25 % by weight of the final composition obtained.

11. Compositions of synthetic substances flameproofed according to one of Claims 8 to 10.